(19) Europäisches Patentamt / European Patent Office / Office européen des brevets

(11) EP 2 523 933 B1

(12) # EUROPÄISCHE PATENTSCHRIFT

(45) Veröffentlichungstag und Bekanntmachung des Hinweises auf die Patenterteilung:
**18.03.2015 Patentblatt 2015/12**

(21) Anmeldenummer: **11700164.4**

(22) Anmeldetag: **10.01.2011**

(51) Int Cl.:
***C07C 209/36*** (2006.01) ***C07C 211/51*** (2006.01)

(86) Internationale Anmeldenummer:
**PCT/EP2011/050233**

(87) Internationale Veröffentlichungsnummer:
**WO 2011/086050 (21.07.2011 Gazette 2011/29)**

(54) **VERFAHREN ZUR HERSTELLUNG VON AROMATISCHEN AMINEN IN DER FLÜSSIGPHASE**

METHOD FOR PRODUCING AROMATIC AMINES IN THE LIQUID PHASE

PROCÉDÉ POUR PRÉPARER DES AMINES AROMATIQUES EN PHASE LIQUIDE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.01.2010 DE 102010004566**

(43) Veröffentlichungstag der Anmeldung:
**21.11.2012 Patentblatt 2012/47**

(73) Patentinhaber: **Bayer Intellectual Property GmbH
40789 Monheim (DE)**

(72) Erfinder:
- **LORENZ, Wolfgang
41540 Dormagen (DE)**
- **PENNEMANN, Bernd
51467 Bergisch Gladbach (DE)**

(74) Vertreter: **BIP Patents
c/o Bayer Intellectual Property GmbH
Creative Campus Monheim
Alfred-Nobel-Straße 10
40789 Monheim (DE)**

(56) Entgegenhaltungen:
**EP-A2- 1 935 871 GB-A- 1 452 466**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 523 933 B1

## Beschreibung

Die Erfindung betrifft ein Verfahren zur Herstellung von aromatischen Aminen in der Flüssigphase durch katalytische Hydrierung der entsprechenden Nitroaromaten in mindestens zwei hintereinandergeschalteten Reaktionsräumen, wobei mindestens ein Reaktionsraum isotherm und mindestens der diesem nachgeschaltete Reaktionsraum adiabat betrieben wird und in bevorzugten Ausführungsformen der adiabate Temperatursprung zur Reaktionskontrolle eingesetzt wird.

Es ist beispielsweise aus EP 0 223 035 A1 bekannt, dass man aromatische Diamine wie z. B. Toluylendiamin (TDA, Diaminotoluol) durch katalytische Hydrierung der entsprechenden aromatischen Dinitroverbindungen herstellen kann. Die Hydrierung wird mit Hilfe von im Reaktionsgemisch dispergierten modifizierten Raney-Nickel-Katalysatoren durchgeführt, die dann durch Filtration oder Sedimentation abgetrennt und gegebenenfalls in den Prozess zurückgeführt werden. Der Schwerpunkt dieser Schrift liegt auf dem Einsatz der modifizierten Raney-NickelKatalysatoren. Die Verwendung von mehreren hintereinandergeschalteten, unterschiedlich (teilweise isotherm, teilweise adiabat) betriebenen Reaktionsräumen, wird dort nicht offenbart. Die in EP 0 223 035 A1 beschriebene Hydrierreaktion ist sehr stark exotherm. Ständige Aufgabe bei der Hydrierung beispielsweise von Dinitrotoluol (DNT) zu Toluylendiamin (TDA) ist es daher, diese Wärme abzuführen. So beschreibt WO 96/11052 A1 eine Reaktionsapparatur für die Durchführung von Schlammphasenhydrierungen unter Verwendung der Reaktionswärme für die Erzeugung von nutzbarem Dampf. Auch dort wird die Verwendung von mehreren hintereinandergeschalteten, unterschiedlich (teilweise isotherm, teilweise adiabat) betriebenen Reaktionsräumen, nicht offenbart.

DE-OS-2 044 657 beschreibt ein Verfahren zur Herstellung von Toluylendiamin durch Hydrierung von Dinitrotoluol bei erhöhtem Druck und erhöhter Temperatur an einem oder zwei hintereinandergeschalteten Reaktoren, bevorzugt Hochdruckrohren, die mit fest angeordneten, Nickel oder Ruthenium enthaltenden Hydrierkatalysatoren bestückt sind. Beim Einsatz zweier Hydrierreaktoren findet im ersten ein Umsatz von etwa 90 % statt. Es wird nicht explizit genannt, ob diese Reaktoren isotherm oder adiabat betrieben werden sollen, nur dass in beiden Reaktoren die gleichen Bedingungen eingehalten werden. Der genannte Vorteil dieses Verfahrens sei der Wegfall der kontinuierlichen Katalysatorabtrennung. Nachteilig ist bei diesem Verfahren, dass eine große Menge des erhaltenen Reaktionsgemisches zwecks Führung im Kreis aus dem Produktgemisch entnommen, Wasser destillativ entfernt und das so gewonnene, wasserfreie rohe Hydriergemisch wieder in die Reaktion zurückgeführt werden muss.

US 3 761 521 beschreibt ein Verfahren zur Hydrierung von aromatischen Nitroverbindungen, bei dem die Hydrierung in einem gerührten Behälter mit innenliegenden Kühlschlangen durchgeführt wird. Mit dem Reaktor ist eine Katalysatorabtrennung mittels Sedimentation verbunden. Infolge der Dichteunterschiede zirkuliert das ausreagierte Reaktionsgemisch vom Reaktor in den Sedimentationsbehälter, wo es in einen katalysatorhaltigen Strom und einen weitgehend von Katalysator befreiten Strom zerlegt wird. Ersterer wird in den Reaktor zurückgeführt, letzterer der Aufarbeitung zugeführt. Verläuft die Reaktion schnell genug, reicht zur kompletten Eduktumsetzung eine Reaktor-/ Sedimentationsbehältereinheit aus. Für langsamere Reaktionssysteme wird empfohlen, zwei oder mehr dieser Systeme hintereinanderzuschalten, was einen hohen apparativen Aufwand erfordert. Auch in dieser Schrift wird die Verwendung von mehreren hintereinandergeschalteten, unterschiedlich (teilweise isotherm, teilweise adiabat) betriebenen Reaktionsräumen, nicht offenbart.

In DE 198 44 901 C1 wird ein Verfahren zur Herstellung von aromatischen Aminen nach dem Sumpfphasenverfahren beschrieben, bei dem die Nitroaromaten über eine Ringleitung mit Löchern in den Reaktor eingespeist werden. Die Ringleitung kann auch über einen außenliegenden Wärmeübertrager gekühlt werden, um die Gefahr einer Überhitzung und damit einer thermischen Zersetzung der Nitroaromaten auszuschließen. Bei diesem Verfahren soll eine besonders gute Verteilung der Nitroaromaten in der Reaktionsmischung erreicht werden. Als geeignete Reaktoren werden beispielsweise Loop-Reaktoren, Blasensäulen, vorzugsweise Rührkessel beschrieben. Auch in dieser Schrift wird die Verwendung von mehreren hintereinandergeschalteten, unterschiedlich (teilweise isotherm, teilweise adiabat) betriebenen Reaktionsräumen, nicht offenbart.

Ein Schlaufenreaktor wird in EP 0 634 391 A1 beschrieben. Hierbei wird ein Gemisch aus Katalysator und DNT, bereits gebildetem Reaktionsprodukt und Wasserstoff mittels eines Ejektors in einem Behälter so gemischt, dass die Reaktion stattfinden kann. Wesentliche weitere Elemente sind eine Zirkulationspumpe, ein Wärmetauscher im Zirkulationsstrom zwecks Abfuhr der Reaktionswärme sowie eine Filtereinheit zur Abtrennung des Produktstroms und Zurückhaltung des suspendierten Katalysators. Auch in dieser Schrift wird die Verwendung von mehreren hintereinandergeschalteten, unterschiedlich (teilweise isotherm, teilweise adiabat) betriebenen Reaktionsräumen, nicht offenbart.

Ein weiteres Verfahren, bei dem ein Schlaufenreaktor eingesetzt wird, beschreibt WO 00/35852 A1. Bevorzugt wird dabei in den Reaktor ein Wärmetauscher integriert, um die Reaktionswärme abzuführen. Zusätzlich zu dem Schleifenreaktor wird ein äußerer Kreislauf beschrieben, wobei vorzugsweise im unteren Bereich des Schleifenreaktors Reaktionsgemisch entnommen wird und mittels eines Förderorgans dem oberen Bereich des Reaktors wieder zugeführt wird. Dieser äußere Kreislauf kann einen zusätzlichen Wärmetauscher enthalten und kann zum Produktaustrag unter Zurückhaltung des Katalysators mittels zum Beispiel Schwerkraftabscheider, Querstromfilter oder Zentrifuge dienen.

Die Reaktionsführung wird als hoch isotherm beschrieben. Zusätzlich kann sich zur Vervollständigung der Umsetzung im äußeren Kreislauf ein Nachreaktor befinden. Der mögliche Typ dieses Nachreaktors wird allgemein als dem Hauptreaktor artgleich oder als Rührkessel oder als Strömungsrohr angegeben. Es wird nicht darauf eingegangen, wie eine erforderliche Nachreaktion erkannt oder von den Reaktionsbedingungen her durchgeführt wird. WO 00/35852 lehrt, dass die Reaktion von Dinitrotoluol zu Toluylendiamin praktisch quantitativ im Hauptreaktor erfolgt und die externe Schlaufenströmung im Wesentlichen reines Toluylendiamin, Wasser und gegebenenfalls Lösungsmittel und Katalysator enthält. Toluylendiamin wird der externen Schlaufenströmung entnommen und der weiteren Aufarbeitung zugeführt. Der Einsatz eines *adiabat* betriebenen Nachreaktors und die Reaktionskontrolle durch Überwachung des adiabaten Temperatursprungs wird in WO 00/35852 nicht gelehrt.

Die Möglichkeit des Auftretens von nicht umgesetzten Nitroverbindungen in einem Reaktionssystem wie in WO 00/35852 wird allerdings in DE 102 06 214 A1 beschrieben. DE 102 06 214 A1 lehrt, dass durch eine Veränderung der Aufgabe der Edukte (Strömungsrichtung zumindest eines Teils des Wasserstoffs und/oder des Nitroaromaten in der flüssigen Reaktionsmischung nach oben) die Umsetzung der Nitroaromaten vollständiger erfolgt. Dies gilt insbesondere für die Hydrierung von DNT. Auch bei dem Verfahren in DE 102 06 214 A1 wird die Reaktionswärme am Ort der Entstehung abgeführt. Der Einsatz eines *adiabat* betriebenen Nachreaktors und die Reaktionskontrolle durch Überwachung des adiabaten Temperatursprungs wird auch in dieser Schrift nicht gelehrt.

Bei allen Verfahren nachteilig ist, dass das erhaltene Reaktionsgemisch neben dem Zielprodukt der aromatischen Amine, dem Koppelprodukt Wasser und den aus den bekannten Nebenreaktionen wie Kernhydrierungen oder hydrogenolytischen Spaltungen resultierenden Verbindungen oder hochmolekularen, teerartigen Produkten auch die als Edukte eingesetzten Nitroaromaten oder Zwischenstufen der Reaktion wie Nitrosoaromaten aufweisen kann. Bekannt ist, dass diese Verbindungen insbesondere in Gegenwart von Aminen und bei erhöhter Temperatur wenig stabil sind und sich explosionsartig zersetzen können. Es muss daher sichergestellt werden, dass die Umsetzung vollständig erfolgt. Üblicherweise wird daher die Reaktion überwacht. Dies kann beispielsweise durch gaschromatographische Analyse des Produktstroms erfolgen, wie in WO 03/066571 A1 beschrieben. Eine andere Vorgehensweise ist die durch Überwachung des in die Reaktion eintretenden Massestroms wie in DE 102 06 214 A1 beschrieben.

DE 10 2005 008 613 A1 beschreibt ein Verfahren zur Herstellung aromatischer Amine, bei dem im Reaktionsprodukt die Gehalte an Nitro- und Nitrosoverbindungen mittels UV-VIS-Absorption bestimmt wird. DE 10 2005 008 613 A1 lehrt, dass bei der katalytischen Hydrierung von Nitroaromaten die Katalysatoren mit der Zeit deaktiviert werden. Je geringer die Aktivität der Katalysatoren ist, umso geringer ist der zu Aminen umgesetzte Anteil der Edukte, so dass der in dem Reaktor verbleibende Anteil an nicht umgesetzten Nitroaromaten steigt. Daher ist eine Überwachung der Katalysatoraktivität notwendig, insbesondere um dem Reaktor eine ausreichende Menge an unverbrauchtem Katalysator zuzuführen. Das Verfahren soll auch zur Dosierung von Nitroaromat und Frischkatalysator zum Reaktorsystem genutzt werden können. Nachteilig an diesem Verfahren ist, dass sich die Spektren von TDA/Wassergemischen mit und ohne Nitro-/Nitrosoverbindungen nur wenig unterscheiden, was aufwendige Kalibrierungen erfordert. Zudem verändern Beläge auf den Messzellen das Messsignal, so dass die Kalibrierung regelmäßig überprüft werden muss. Auch Gasblasen oder Katalysatorpartikel stören die Messung, so dass eine aufwendige Probenvorbereitung erforderlich wird, die, neben dem Zeitverlust zwischen Auftreten und Erfassen des Effektes, nicht nur hohe Kosten, sondern auch eine geringe Zuverlässigkeit der Gesamtanordnung bewirkt.

Mit der Verbesserung der Stoffaustauschvorgänge bei heterogen katalysierten Hydrierreaktionen, wie zum Beispiel der Herstellung aromatischer Amine aus den entsprechenden Nitroverbindungen, beschäftigt sich WO 02/062729 A2. Durch einen bestimmten Inertgasanteil im Hydrierwasserstoff soll eine höhere Wasserstoffsättigung der Flüssigphase im Reaktor erreicht werden, was einer erhöhten Alterung des Hydrierkatalysators und einer unzureichenden Selektivität der Reaktion vorbeugen soll. Ziel auch dieser Veröffentlichung ist es letztendlich, für eine möglichst vollständige Umsetzung der Nitroverbindung zum Amin im Hydrierreaktor zu sorgen. Nicht behandelt wird die Kontrolle der Hydrierqualität, also Auswirkung einer auftretenden Katalysatoralterung oder einer unzureichenden Selektivität, und wie mit solchen Situationen umgegangen werden kann.

Grundgedanke bei den oben aufgeführten Verfahren ist es, entweder die Reaktionswärme mittels Wärmetauschern am Ort der Entstehung abzuführen oder in einem ausreichend großen Produktstrom aufzunehmen und an einem anderen Ort mittels Wärmeaustauschern so abzuführen, dass unzulässig hohe Temperaturen vermieden werden. Ziel der genannten Verfahren ist es daher regelmäßig, die Anströmung von zur Wärmeabfuhr verwendeten Wärmeaustauschern und die Durchmischung der in den für die Reaktion verwendeten Apparaten so weit zu steigern, dass die Temperaturunterschiede im Reaktionsraum möglichst gering sind. Für eine rasche Hydrierung wesentlich ist zudem das Vorhandensein von ausreichenden Gehalten an Wasserstoff im Reaktionsraum, was den Eintrag von Gasblasen in den Reaktionsraum erfordert und beispielsweise mittels Begasungsrührer, Kreisgasverdichter oder Schlaufenreaktoren erreicht werden kann. Diese Begasung führt ebenfalls zu einer intensiven Durchmischung des Reaktionsraums. Dabei ist offensichtlich, dass sich durch diese Maßnahmen die Verweilzeitverteilung dem eines ideal durchmischten Rührkessels annähert, was das Auftreten von Edukten im Produktstrom begünstigt und das daher wie oben ausgeführt unerwünscht ist.

EP-A-1 935 871 beschreibt ein Verfahren zur Nitroaromatenhydrierung in der Flüssigphase, bei dem mehrere

gleiche oder eine Kombination unterschiedlicher geeigneter Reaktionsapparate eingesetzt werden können. Die Kombination eines isothermen Reaktionsraums und mindestens eines nachgeschalteten adiabatischen Reaktionsraums wird dorthin nicht offenbart.

Mit dem Einsatz von hintereinandergeschalteten isotherm und adiabat betriebenen Reaktoren mit Festbettkatalysatoren in der *Gasphasen*hydrierung von Nitroaromaten befassen sich EP 1 524 259 A1 (bevorzugt Nitrobenzol als Edukt) und GB 1 452 466 (ausschließlich Nitrobenzol als Edukt). Das Problem des Explosionsschutzes stellt sich bei Gasphasenhydrierungen längst nicht in dem Maße wie oben für Flüssigphasenhydrierungen (insbesondere von *Di*nitroaromaten) angeführt. So wird die Möglichkeit, den Temperatursprung in einem adiabat betriebenen Reaktionsraum zur Überwachung des Umsatzes an Nitro- bzw. Nitrosoaromaten heranzuziehen in keiner der beiden Schriften gelehrt. In EP 1 524 259 A1 geht es nur darum, die Betriebszeit eines isotherm betriebenen Hauptreaktors (Rohrbündelreaktors) durch einen nachgeschalteten, adiabat betriebenen Reaktor mit monolithischem Katalysator bei Verdünnung des Katalysatorbettes zur Dämpfung der Reaktivität des Hauptreaktors zu verlängern (S. 4, Z. 32 - 33 und Z. 40). Der Einsatz von Monolithen ist dabei erfindungswesentlich, um unzulässig hohe Differenzdrücke zu vermeiden. In GB 1 452 466 schließlich dient die Kombination aus isothermer und adiabater Fahrweise der besseren Temperaturkontrolle (S. 3, Z. 86 - 94). Als vorteilhaft bei diesem Verfahren werden die Erhöhung der Raum-Zeit-Ausbeute und der Standzeit genannt. Die verfahrenstechnischen Herausforderungen sind bei der Gasphasenhydrierung völlig andere als bei der Flüssigphasenhydrierung, sodass die beiden Prozesse kaum oder gar nicht vergleichbar sind.

**Aufgabe** der vorliegenden Erfindung ist es daher, ein Verfahren zur Hydrierung von Nitroaromaten, insbesondere Dinitroaromaten, zu den entsprechenden aromatischen Aminen, insbesondere Diaminen, in der Flüssigphase bereitzustellen, bei dem der Restgehalt an Nitroaromaten (d. h. Ausgangsnitroaromaten und im Falle von Dinitroverbindungen als Eduke zusätzlich die intermediär auftretenden aromatischen Mononitro-Monoamin-Verbindungen) und ggf. der als Zwischenverbindungen auftretenden Nitrosoaromaten (d. h. alle denkbaren, mindestens eine Nitrosogruppe enthaltenden Zwischenstufen) im erhaltenen rohen Produktgemisch minimiert wird und das eine einfache und zuverlässige Überwachung des Reaktionsverlaufs (Vollständigkeit des Reaktion der Nitroaromaten zu den aromatischen Aminen) ermöglicht.

**Gegenstand** der Erfindung ist also ein Verfahren zur Herstellung von aromatischen Aminen der Formel

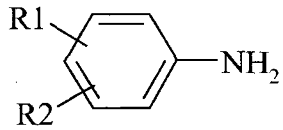

(I)

in der R1 und R2 unabhängig voneinander Wasserstoff, Methyl oder Ethyl bedeuten, wobei R1 zusätzlich $NH_2$ bedeuten kann, durch Hydrierung in Gegenwart eines Katalysators von Nitroaromaten der Formel

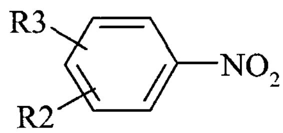

(II)

in der R2 und R3 unabhängig voneinander Wasserstoff, Methyl oder Ethyl bedeuten, wobei R3 zusätzlich $NO_2$ bedeuten kann, in der Flüssigphase in mindestens zwei hintereinandergeschalteten, in einem Reaktor oder in mehreren Reaktoren angeordneten, Reaktionsräumen, wobei

mindestens ein Reaktionsraum isotherm und mindestens der diesem nachgeschaltete Reaktionsraum adiabat betrieben wird.

Die *Hydrierung* des Nitroaromaten zum entsprechenden Amin erfolgt dabei mit Wasserstoff oder Gemischen aus Wasserstoff und inerten Gasen als Hydrierreagenz. Als *Katalysatoren* kommen dabei alle für katalytische Hydrierungen üblichen in Frage. Bevorzugt werden Katalysatoren, die Edelmetalle wie Pt, Pd, Rh, Ru oder Buntmetalle wie Ni, Co oder Cu oder deren Mischungen enthalten, eingesetzt. Besonders bevorzugt werden Katalysatoren, die Pt, Pd, Ni oder Cu enthalten, eingesetzt, und zwar als Suspension in Wasser. Im Falle von Edelmetallkatalysatoren werden diese auf Träger wie bspw. Aktivkohle, $SiO_2$ oder $Al_2O_3$ aufgebracht, im Fall von Ni-Katalysatoren kann auch Raney-Ni eingesetzt werden. Die Konzentration von Katalysator im Reaktionsraum liegt bevorzugt zwischen 0,01 Massen-% und < 20 Massen-%, bevorzugt zwischen 0,5 Massen-% und 10 Massen-%, bezogen auf die Gesamtmasse des Reaktionsgemisches im Reaktionsraum. Werden Gemische aus Wasserstoff und inerten Gasen eingesetzt, so sind bevorzugte Inertgase Ammoniak, Edelgase und/oder Stickstoff. Wasserstoff bzw. das Gemisch aus Wasserstoff und inerten Gasen wird bevorzugt so aufgegeben, dass sich im Reaktionsraum ein konstanter Druck einstellt, d. h. mit zunehmendem

Reaktionsfortschritt (und damit Wasserstoffverbrauch) wird die Zufuhr von frischem Hydrierreagenz erhöht. Im Falle der Verwendung eines Gemisches aus Wasserstoff und inerten Gasen als Hydrierreagenz wird bevorzugt das Verhältnis aus Wasserstoff und Inertgas im zugeführten Hydrierreagenz sukzessive erhöht, um den Reaktorinhalt nicht an Wasserstoff verarmen zu lassen.

Unter *Reaktionsraum* wird dabei der Raum verstanden, in welchem die Vorraussetzungen für eine Reaktion in der Flüssigphase von Nitroaromat (bzw. Zwischenprodukten) mit Wasserstoff zum gewünschten aromatischen Amin gegeben sind. Der Reaktionsraum befindet sich in einer technischen Vorrichtung zur Durchführung von chemischen Reaktionen, dem Reaktor. Reaktionsraum und Reaktor können, je nach Konstruktion, auch identisch sein (z. B. bei Blasensäulenreaktoren). Der Reaktionsraum kann auch nur einen Teil des Reaktors umfassen. Wenn etwa nur im unteren Bereich eines Reaktors eine Flüssigphase vorliegt, so gehört die darüber liegende Gasphase nicht mehr zu erfindungsgemäßen Reaktionsraum, ungeachtet der Tatsache, dass infolge des Dampfdrucks des Nitroaromaten möglicherweise ein - untergeordneter - Anteil des Nitroaromaten in die Gasphase eintritt und dort reagiert. Ein Reaktor kann auch mehrere Reaktionsräume enthalten. Die Reaktionsräume können sich in einem oder in verschiedenen Reaktoren befinden. Bevorzugte Reaktoren für das erfindungsgemäße Verfahren sind gerührte Kessel, Schlaufenreaktoren, Strömungsrohre, Blasensäulenreaktoren oder Jet-Reaktoren.

Die Reaktionsräume sind im erfindungsgemäßen Verfahren *hintereinandergeschaltet*, d. h. die Produktmischung aus einem Reaktionsraum wird in den folgenden Reaktionsraum als Eduktmischung eingeleitet. Es ist möglich, jedoch nicht unbedingt zwingend erforderlich, in die nachgeschalteten Reaktionsräume zusätzlich frischen Wasserstoff oder ein Gemisch aus Wasserstoff und Inertgasen und ggf. frischen Katalysator einzuleiten. Im erfindungsgemäßen Verfahren wird der frische Nitroaromat nur in einen Reaktionsraum eingeleitet; dieser wird als der *in Anströmrichtung des Nitroaromaten erste* bezeichnet und bevorzugt isotherm betrieben. Alle nachfolgenden Reaktionsräume werden nur mit Nitroaromaten, welcher im jeweils vorigen Reaktionsraum nicht umgesetzt wurde, belastet. Demnach werden bei Vollumsatz im in Anströmrichtung des Nitroaromaten ersten Reaktionsraum die nachfolgenden überhaupt nicht mit Nitroaromaten belastet.

Es ist auch vorstellbar, mehr als zwei Reaktionsräume in Reihe zu schalten, also bspw. Kaskaden wie "isotherm - adiabat - adiabat" (vgl. Figur 4), "isotherm - isotherm - adiabat - adiabat" oder andere Kombinationen. Es werden bevorzugt maximal 10 Reaktionsräume hintereinandergeschaltet, besonders bevorzugt maximal 5, ganz besonders bevorzugt maximal 3, außerordentlich ganz besonders bevorzugt maximal 2. Bevorzugt werden der in Anströmrichtung des Nitroaromaten erste Reaktionsraum einer Kaskade isotherm und der letzte Reaktionsraum dieser Kaskade adiabat betrieben. Die Erfindung wird im Folgenden an der Ausführungsform mit zwei hintereinandergeschalteten Reaktionsräumen näher erläutert. Es ist dem Fachmann ein Leichtes, die Angaben erforderlichenfalls auf Systeme mit mehr als zwei Reaktionsräumen zu übertragen.

*Isotherm* bedeutet im Rahmen der vorliegenden Erfindung, dass mindestens der überwiegende Teil der durch die Reaktion freigesetzten Wärme durch technische Vorrichtungen abgeführt wird. Bevorzugt wird die Reaktionswärme vollständig durch technische Vorrichtungen abgeführt. Solche Vorrichtungen können sein: Rohrbündelwärmeaustauscher, Rohrschlangen, Plattenwärmeaustauscher, Fieldrohre, U-Rohr-Wärmeaustauscher. Diese Vorrichtungen sind bevorzugt im isotherm betriebenen Reaktionsraum angebracht. Alternativ können sie auch außerhalb des isotherm betriebenen Reaktionsraums liegen, wenn durch hinreichend große Kreislaufströme eine ausreichende Isothermie (d. h. höchstens ein vernachlässigbarer Temperaturanstieg) im Reaktionsraum sichergestellt ist.

Dementsprechend bedeutet *adiabat* im Rahmen der vorliegenden Erfindung, dass die Reaktionswärme im adiabat betriebenen Reaktionsraum nicht durch technische Vorrichtungen abgeführt wird. Bei adiabater Betriebsweise kann der Reaktionsraum in besonderer Weise gegen Wärmeverluste isoliert werden. Wenn Wärmeverluste vernachlässigbar sind, spiegelt sich die Reaktionsenthalpie in der Temperaturdifferenz zwischen Eingangs- und Austrittsstrom quantitativ wider.

Umsetzung *in der Flüssigphase* bedeutet, dass die Reaktion bei solchen physikalischen Bedingungen, insbesondere von Druck und Temperatur, durchgeführt wird, bei denen der Nitroaromat, Zwischenprodukte und das aromatische Amin flüssig vorliegen. Bevorzugt werden absolute Drücke zwischen 6 bar und 101 bar, bevorzugt zwischen 10 bar und 30 bar sowie Temperaturen zwischen 50 °C und 250 °C, bevorzugt zwischen 100 °C und 200 °C, besonders bevorzugt 120 °C und 195 °C, in allen Reaktionsräumen eingehalten. Innerhalb dieser Bereiche können Druck und Temperatur in den einzelnen Reaktionsräumen unterschiedlich sein.

Bevorzugt ist der Druck im adiabat betriebenen Reaktionsraum gleich dem Druck im diesem vorangehenden isotherm betriebenen Reaktionsraum. Dabei wird der Druck bevorzugt im Kopfbereich des Reaktors gemessen. In bestimmten Ausführungsformen des erfindungsgemäßen Verfahrens liegt daher die Messstelle für den Druck außerhalb des eigentlichen Reaktionsraums (bspw. in der Gasphase oberhalb der den Reaktionsraum darstellenden Flüssigphase). Dieser so gemessene Druck ist nicht notwendigerweise identisch mit dem Druck im Reaktionsraum selbst, jedoch in jedem Fall für diesen charakteristisch, sodass er für relative Vergleiche zwischen den Drücken in verschiedenen Reaktionsräumen einen geeigneten Bezugspunkt darstellt. Der Druck im adiabat betriebenen Reaktionsraum kann auch geringfügig unter dem Druck im isotherm betriebenen Reaktionsraum liegen. Wird jedoch der Druck im adiabat betriebenen Reaktionsraum gegenüber dem Druck im isotherm betriebenen Reaktionsraum zu stark abgesenkt, tritt eine Entspannungsverdampfung des Reaktionsgemisches im Zulauf des adiabat betriebenen Reaktionsraums ein, die zu einer deutlichen Verringerung des Anteils an gelöstem und/oder dispergiertem Wasserstoff in der Flüssigphase führt. Für das erfindungsgemäße Verfahren ist daher das Verhältnis aus dem Druck in einem beliebigen adiabat betriebenen Reaktionsraum zum Druck im diesen jeweils vorangehenden isotherm betriebenen Reaktionsraum größer als 0,5, bevorzugt größer als 0,7 und besonders bevorzugt größer als 0,9.

Bevorzugt entspricht die mittels geeigneter Vorrichtungen zur Wärmeabfuhr im isotherm betriebenen Reaktionsraum eingestellte Temperatur der Eintrittstemperatur des diesem nachgeschalteten adiabat betriebenen Reaktionsraums.

Optional können unter den Reaktionsbedingungen inerte Lösungsmittel eingesetzt werden, wie z. B. Alkohole wie Methanol, Propanol, Isopropanol oder Ether wie Dixoan, Tetrahydrofuran. Um die Wirtschaftlichkeit des Verfahrens zu erhöhen, ist in der Regel eine niedrige Lösungsmittelkonzentration vorteilhaft; üblicherweise wird diese zwischen 1 Massen-% und < 50 Massen-%, bevorzugt zwischen 20 Massen-% und < 35 Massen-%, bezogen auf die Gesamtmasse der Flüssigphase, liegen.

Bevorzugt beträgt der Umsatz an Nitroaromaten und ggf. der als Zwischenverbindungen auftretenden Nitrosoaromaten im in Anströmrichtung des Nitroaromaten ersten Reaktionsraum zu Beginn der Hydrierung, wenn der Katalysator noch frisch und hochaktiv ist, 100 %. Mit fortschreitender Katalysatordesaktivierung wird der Umsatz in diesem Reaktionsraum, wenn keine Gegenmaßnahmen eingeleitet werden, immer weiter sinken. Die nichtumgesetzten Anteile an Nitro- und ggf. Nitrosoaromat werden im erfindungsgemäßen Verfahren bevorzugt im adiabat betriebenen Reaktionsraum weiter umgesetzt, besonders bevorzugt vollständig.

Das erfindungsgemäße Verfahren eignet sich besonders für die Hydrierung von DNT (in Formel II: R2 = Methyl und R3 = $NO_2$) zu TDA (in Formel I: R1 = $NH_2$ und R2 = Methyl). Im großtechnischen Prozess treten alle Isomere auf, hauptsächlich jedoch 2,4-TDA und 2,6-TDA.

Das erfindungsgemäße Verfahren wird bevorzugt kontinuierlich ausgeführt. Eine diskontinuierliche Fahrweise ist jedoch nicht ausgeschlossen.

In einer bevorzugten Ausführungsform der vorliegenden Erfindung wird einem isotherm betriebenen Reaktionsraum ein adiabat betriebener nachgeschaltet, wobei die aus dem isotherm betriebenen Reaktionsraum erhaltene, suspendierten Katalysator enthaltende, Produktmischung in den adiabat betriebenen Reaktionsraum eingeleitet und nach weiterer, bevorzugt vollständiger Umsetzung in zwei Ströme aufgeteilt wird, von denen einer von Katalysator weitestgehend befreit und der weiteren Aufarbeitung zugeführt wird. Für die Abtrennung von Katalysator eignen sich Schwerkraftabscheidung, beispielsweise Settler sowie Filtration, beispielsweise Querstromfilter. Der so gewonnene Katalysator kann, ggf. nach Aufarbeitungsprozessen, in den isotherm betriebenen Reaktionsraum zurückgeführt werden. Gegebenenfalls kann auch noch frischer Katalysator zugeführt werden. Der andere, katalysatorhaltige Strom wird in den isotherm betriebenen Reaktionsraum zurückgeführt. Die Abtrennung von Reaktionswasser aus diesem zurückgeführten Strom ist möglich, jedoch nicht bevorzugt.

Der Transport des Reaktionsgemisches aus dem isotherm betriebenen Reaktionsraum in den adiabat betriebenen Reaktionsraum kann bei dieser Ausführungsform auf verschiedene Weisen erfolgen, beispielsweise durch Pumpen oder unter Einfluss der Schwerkraft mittels Überlauf oder Dichteunterschiede. Für den Rücktransport des katalysatorhaltigen Stromes gilt Entsprechendes.

In einer weiteren Ausführungsform wird ein Reaktor durch geeignete Strömungsführung in zwei Reaktionsräume aufgeteilt (siehe Figur 4). Dabei wird der in Anströmrichtung des Nitroaromaten erste Reaktionsraum mit einer Vorrichtung zur Wärmeabfuhr versehen ("isothermer Reaktionsraum") und der zweite nicht ("adiabater Reaktionsraum"). Der Wasserstoff bzw. das Gemisch aus Wasserstoff und inerten Gasen wird nur in den "isothermen Reaktionsraum" eingetragen.

Die erfindungsgemäße Hintereinanderschaltung eines isotherm betriebenen und eines adiabat betriebenen Reaktionsraums macht sich die Vorteile beider Verfahrensführungen zu Nutze und hebt sich dadurch in besonderer Weise vorteilhaft vom Stand der Technik ab. So erfolgt der größte Teil des Umsatzes (bevorzugt mehr als 95 %, besonders bevorzugt mehr als 98 %, ganz besonders bevorzugt mehr als 99 %) an Ausgangsnitroaromat und allen ggf. auftretenden Zwischenprodukten im isotherm betriebenen Reaktionsraum unter schonenden Bedingungen, die ein Höchstmaß an Sicherheit gewährleisten. Die Umsetzung des restlichen Nitroaromaten und allen restlichen ggf. auftretenden Zwischenprodukten unter adiabaten Bedingungen macht sich einerseits zu Nutze, dass durch die höheren Temperaturen (keine Wärmeabfuhr!) im adiabat betriebenen Reaktionsraum höhere Umsätze möglich werden (was, da nur noch ein kleiner Teil des Ausgangsnitroaromaten und allen ggf. auftretenden Zwischenprodukten umgesetzt werden muss, gefahrlos möglich ist) und bietet andererseits noch zusätzlich den Vorteil der einfachen Überwachung des Reaktionsverlaufs, insbesondere der Vollständigkeit der Hydrierreaktion von Nitroaromaten zu aromatischem Amin, durch Kontrolle einer geeigneten, für den im adiabat betriebenen Reaktionsraum durchgeführten Teil der Hydrierung charakteristischen, Kenngröße. Geeignete Kenngrößen können unter anderem dabei sein: Farbe, Temperatur, die Konzentration an Nitro- und Nitrosogruppen enthaltenden Verbindungen, ermittelt durch chemische Analyse, vorzugsweise durch Gaschromatographie, oder durch spektroskopische Analyse, vorzugsweise durch UV-VIS-Spektroskopie. Werden mehrere adiabat betriebene Reaktionsräume eingesetzt, so kann die Kontrolle dieser geeigneten Kenngröße für einen, mehrere oder alle adiabat betriebenen Reaktionsräume durchgeführt, werden. In einer Kaskade aus mehr als zwei Reaktionsräumen wird bevorzugt mindestens der letzte Reaktionsraum adiabat betrieben und mindestens für diesen die charakteristische Kenngröße kontrolliert. Prinzipiell können auch verschiedene Kenngrößen kontrolliert werden, bspw. Temperatur und Farbe.

Bevorzugt wird für jede Kenngröße durch Vorversuche ein kritischer Wert ermittelt, etwa im Falle der Temperatur eine nicht zu überschreitende kritische Temperatur, wie weiter unten noch im Detail ausgeführt wird. Bei Erreichen oder Überschreiten dieses zuvor definierten kritischen Wertes dieser Kenngröße kann dann (mindestens) ein Prozessparameter so geändert werden, dass diese Kenngröße wieder unter den kritischen Wert sinkt, wie weiter unten noch im Detail erläutert wird. Der kritische Wert für die Konzentration an Nitro- und Nitrosogruppen enthaltenden Verbindungen ist stark von den konkreten Randbedingungen einer Produktionsanlage abhängig (z. B. Temperatur, Druck, Art des zu hydrierenden Nitroaromaten und verwendeter Katalysator). Er kann beispielsweise bevorzugt bei Werten bis kleiner als 5,0 Massen-%, besonders bevorzugt bis kleiner als 2,0 Massen-% und ganz besonders bevorzugt bis kleiner als 0,5 Massen-%, jeweils bezogen auf die Gesamtmasse des Produktaustrittstroms des adiabat betriebenen Reaktionsraumes, liegen.

Das erfindungsgemäße Verfahren ermöglicht in bevorzugten Ausführungsformen die Ermittlung eines mindestens qualitativen, bevorzugt quantitativen Zusammenhanges zwischen bestimmten Kenngrößen und dem Gehalt an Nitro-/Nitrosoaromaten im Eintritt in den und/oder Austritt aus dem adiabat betriebenen Reaktionsraum durch Testversuche oder durch ingenieurtechnische Berechnungen. Während der eigentlichen Produktion kann dann durch Kontrolle einer oder mehrerer dieser Kenngrößen festgestellt werden, wann mindestens ein Prozessparameter geändert werden muss, um den Umsatz wieder zu steigern. In diesem Zusammenhang ist insbesondere die Temperatur die Kenngröße der Wahl, wie weiter unten noch im Detail ausgeführt wird. Die Kenngröße Farbe entzieht sich einer einfachen quantitativen Beschreibung. Erfahrene Anlagenbetreiber können jedoch bereits auf Grund von Farbbeobachtungen (beispielsweise in im Produktaustrittsstrom aus dem adiabat betriebenen Reaktionsraum angeordneten Probenahmestellen) zumindest qualitative Rückschlüsse auf den Reaktionsverlauf ziehen.

Zum Zwecke der Quantifizierung des Zusammenhanges zwischen bestimmten geeigneten Kenngrößen und dem Gehalt an Nitro-/Nitrosoaromaten im Eintritt in den und/oder Austritt aus dem adiabat betriebenen Reaktionsraum kann es in bestimmten Fällen zweckmäßig sein, eine Kalibrierkurve, unter Umständen mehrere Kalibrierkurven für verschiedene Bereiche von Druck und Temperatur, zu erstellen. Wird als Bezugsgröße der Gehalt an Nitro-/Nitrosoaromaten im Eintritt in den adiabat betriebenen Reaktionsraum gewählt, so wird, abhängig von der Leistungsfähigkeit des adiabat betriebenen Reaktionsraums, rechtzeitig mindestens ein Prozessparameter verändert, um sicherzustellen, dass die in den adiabat betriebenen Reaktionsraum eintretende Menge an Nitro-/Nitrosoaromaten möglichst vollständig umgesetzt werden kann.

Beispielsweise kann die im adiabat betriebenen Reaktionsraum oder im Produktaustritt aus dem adiabat betriebenen Reaktionsraum gemessene Temperatur $T_{adiabat}$ eine geeignete Kenngröße sein. Der kritische Wert der Kenngröße $T_{adiabat}$, $T_{adiabat\ krit.}$, liegt - abhängig von den genauen sonstigen Prozessbedingungen und der Art des zu hydrierenden Nitroaromaten - bevorzugt bei Werten zwischen 200 °C und 250 °C.

Das erfindungsgemäße Verfahren ermöglicht eine Überwachung des Reaktionsverlaufs (Vollständigkeit der Hydrierreaktion von Nitroaromaten zu aromatischem Amin) insbesondere auch durch Messung einer Temperaturdifferenz $\Delta T_{adiabat}$ (adiabater Temperatursprung) zwischen mindestens einem der adiabat betriebenen Reaktionsräume und dem diesen jeweils vorangehenden isotherm betriebenen Reaktionsraum. Diese Art der Reaktionskontrolle, die bei rein isothermer Reaktionsführung nicht möglich ist, ist besonders bevorzugt.

Die Temperaturdifferenz $\Delta T_{adiabat}$ wird bevorzugt ermittelt durch Messen der Temperatur $T_{adiabat}$

im Produktaustritt aus dem jeweiligen adiabat betriebenen Reaktionsraum oder im jeweiligen adiabat betriebenen Reaktionsraum selbst

und durch Messen der Temperatur $T_{isotherm}$

im jeweils vorangehenden isotherm betriebenen Reaktionsraum oder im Eintritt in den jeweiligen adiabat betriebenen Reaktionsraum

und anschließende Differenzbildung gemäß

$$\Delta T_{adiabat} = T_{adiabat} - T_{isotherm}.$$

Wenn die Temperatur im Produktaustritt aus dem adiabat betriebenen Reaktionsraum zur Bestimmung von $T_{adiabat}$ herangezogen wird, muss die Messung nicht notwendigerweise *unmittelbar* nach Verlassen des adiabat betriebenen Reaktionsraums erfolgen. Die Temperaturmessstelle kann auch bspw. hinter der Katalysatorabtrennung liegen. Es muss lediglich sichergestellt sein, dass die Messstelle zuverlässige Aussagen über die Wärmeentwicklung im adiabat betriebenen Reaktionsraum gestattet.

Enthält eine Kaskade aus Reaktionsräumen mehr als zwei adiabat betriebene, so können, wenn auch nicht bevorzugt, mehrere solcher adiabater Temperatursprünge gemessen werden. Bspw. können in einer Reaktionsraumkaskade des Typs ,,isotherm (1) - adiabat (1) - isotherm (2) - adiabat (2)" zwei Temperaturdifferenzen, $\Delta T_{adiabat}$ (1) = $T_{adiabat}$ (1) - $T_{isotherm}$ (1) und $\Delta T_{adiabat}$ (2) = Tadiabat (2)-$T_{isotherm}$ (2), ermittelt werden. Sind zwei adiabat betriebene Reaktionsräume unmittelbar hintereinandergeschaltet, also bspw. "isotherm - adiabat (1) - adiabat (2)", so wird bevorzugt die Temperaturdifferenz $T_{adiabat}$ (2) - $T_{isotherm}$ ermittelt. Der "jeweils vorangehende" isotherm betriebene Reaktionsraum ist in diesem Fall nicht der unmittelbar vorangehende Reaktionsraum (der ja auch adiabat betrieben wird).

Die Temperaturmessung erfolgt mittels dem Fachmann bekannter Vorrichtungen, wie Thermoelemente oder Widerstands-, Halbleiter- oder Infrarotthermometer. Eine geeignete Stelle zur Bestimmung von $T_{isotherm}$ im isotherm betriebenen Reaktionsraum ist bspw. bei einem gerührten Reaktor die Umgebung in der Nähe des Rührers oder bevorzugt der Bereich der den Rührer umgebenden Reaktorwand. Eine so innerhalb des isotherm betriebenen Reaktionsraums gemessene Temperatur ist i. A. - wenn geringfügige, unvermeidbare Wärmeverluste vernachlässigt werden- identisch mit der Eintrittstemperatur in den adiabat betriebenen Reaktionsraum. Eine geeignete Stelle zur Bestimmung von $T_{adiabat}$ im adiabat betriebenen Reaktionsraum ist nahe der Reaktorwand im Bereich des Austritts aus dem adiabat betriebenen Reaktionsraum. Die Temperatur im Produktaustritt aus dem adiabat betriebenen Reaktionsraum ist im Allgemeinen identisch mit der mittleren infolge der Wärmeentwicklung in diesem adiabat betriebenen Reaktionsraum erreichten absoluten Temperatur (= adiabate Sprungtemperatur; zu unterscheiden vom adiabatem Temperatursprung $\Delta T_{adiabat}$). Wesentlich ist, dass die Messstellen so gewählt werden, dass sie repräsentativ sind für die mittlere adiabate Sprungtemperatur im adiabat betriebenen Reaktionsraum und für die mittlere Temperatur im isotherm betriebenen Reaktionsraum.

Bevorzugt wird durch Testversuche ein quantitativer Zusammenhang zwischen $\Delta T_{adiabat}$ und dem Gehalt an der Summe aus Nitroaromaten und ggf. der als Zwischenverbindungen auftretenden Nitrosoaromaten im Produktaustritt des adiabat betriebenen Reaktionsraums ermittelt. Beispielsweise kann durch wenige Versuche eine Kalibrierkurve ermittelt werden, die eine Funktion

$$\Delta T_{adiabat} = \Delta T_{adiabat}\,(c_{\Sigma\{\text{Nitro-/Nitrosoaromaten, Austritt adiabat}\}});\ c = \text{Konzentration, bevorzugt in Massen-\%}$$

darstellt. $\Delta T_{adiabat}$ ist darüber hinaus natürlich von den im Einzelnen herrschenden Absoluttemperaturen und -drücken abhängig.

Bevorzugt wird auch eine maximal zulässige Temperaturdifferenz, im Folgenden als $\Delta T_{adiabat, max.}$ bezeichnet, ermittelt. Diese entspricht derjenigen Temperaturdifferenz, die mit einem sowohl unter Qualitäts- als auch Explosionsschutzgesichtspunkten gerade noch akzeptablen Gehalt an der Summe aus Nitroaromaten und ggf. der als Zwischenverbindungen auftretenden Nitrosoaromaten im Produktaustritt des adiabat betriebenen Reaktionsraumes korrespondiert. Ein solcher maximal tolerierbarer Gehalt liegt bevorzugt bei Werten bis zu 5,0 Massen-%, besonders bevorzugt bis zu 2,0 Massen-% und ganz besonders bevorzugt bis zu 0,5 Massen-%, jeweils bezogen auf die Gesamtmasse des Produktaustrittstroms. Bevorzugt wird weiterhin ein Wert von $\Delta T_{adiabat}$ ermittelt, der so weit unterhalb von $\Delta T_{adiabat, max.}$ liegt, dass durch Änderung mindestens eines Prozessparameters das Erreichen von $\Delta T_{adiabat, max.}$ rechtzeitig verhindert werden kann. Dieser Wert ist der kritische Wert der Kenngröße $\Delta T_{adiabat}$ und wird als $\Delta T_{adiabat, krit.}$ bezeichnet.

Die Bestimmung von $\Delta T_{adiabat, max}$ und $\Delta T_{adiabat, krit.}$ kann einerseits durch Versuche geschehen, bei denen sich eine ausreichende Menge Edukt im Zulauf zum adiabat betriebenen Reaktionsraum befindet oder andererseits durch ingenieurtechnische Rechnungen zum Temperaturverlauf im adiabat betriebenen Reaktionsraum. Die im normalen Produktionsbetrieb dann jeweils gemessene Temperaturdifferenz $\Delta T_{adiabat}$ wird mit der so erhaltenen maximalen Temperaturdifferenz $\Delta T_{adiabat, max.}$ und/oder $\Delta T_{adiabat, krit.}$ verglichen.

Ist $\Delta T_{adiabat} = 0$, so findet, *sofern Wärmeverluste vernachlässigt werden können,* die Reaktion vollständig im isotherm betriebenen Reaktionsraum statt, d. h. der Umsatz an Nitroaromaten und ggf. der als Zwischenverbindungen auftretenden Nitrosoaromaten im isotherm betriebenen Reaktionsraum beträgt 100 %. Ein $\Delta T_{adiabat} < 0$ deutet auf Wärmeverluste infolge nicht perfekter Isolierung hin. Ein $\Delta T_{adiabat} > 0$ deutet auf unvollständigen Umsatz im isotherm betriebenen Reaktionsraum hin.

Bei welchem Wert von $\Delta T_{adiabat}$ im Einzelfall die Änderung mindestens eines Prozessparameters erforderlich ist (d. h. wann $\Delta T_{adiabat}$ gleich $\Delta T_{adiabat, krit.}$ wird), hängt von vielen Faktoren ab. Der Bereich, innerhalb dessen $\Delta T_{adiabat}$ gleich $\Delta T_{adiabat, krit.}$ wird, kann im Allgemeinen Werte zwischen -10 K und +8 K, bevorzugt zwischen -8 K und +5 K,

besonders bevorzugt zwischen -5 K und +3 K annehmen.

Ist eine Produktionsanlage sehr gut isoliert, sodass bei Vollumsatz im isotherm betriebenen Reaktionsraum tatsächlich $\Delta T_{adiabat} = 0$ gilt, so zeigt ein Wert von $\Delta T_{adiabat} \leq 0$ verlässlich an, dass im adiabat betriebenen Reaktionsraum keine Hydrierreaktion stattfindet. In einem solchen Fall sind Werte von $\Delta T_{adiabat}$, die nur geringfügig größer als Null sind, im Allgemeinen noch unkritisch. In besonders bevorzugten Ausführungsformen beträgt der kritische Wert von $\Delta T_{adiabat.}$ ($\Delta T_{adiabat,\ krit.}$) +3 K.

Andererseits kann im Falle einer unzulänglich isolierten Produktionsanlage ein Wert von $\Delta T_{adjabat} \leq 0$ *fälschlicherweise vortäuschen*, dass im adiabat betriebenen Reaktionsraum keine Reaktion stattfindet. In einem solchen Fall muss unter Umständen selbst bei Werten von $\Delta T_{adiabat} \leq 0$ mindestens ein Prozessparameter geändert werden. So ist es in besonders bevorzugten Ausführungsformen sinnvoll, bereits bei Werten von $\Delta T_{adiabat} \geq -5$ K (d. h. $\Delta T_{adiabat,\ krit.}$ beträgt -5 K) mindestens einen Prozessparameter zu ändern.

Deutet die Kontrolle einer oder mehrerer der vorstehend genannten geeigneten, für den im adiabat betriebenen Reaktionsraum durchgeführten Teil der Hydrierung charakteristischen, Kenngrößen auf unvollständigen Umsatz im isotherm betriebenen Reaktionsraum hin, so gibt es mehrere Möglichkeiten:

a) Fortführen der Reaktion unter konstanten Bedingungen, bis bei Erreichen eines maximal zulässigen Wertes der jeweiligen Kenngröße (z. B. bei $\Delta T_{adiabat} = \Delta T_{adiabat,\ max}$) die Reaktion abgebrochen wird (Auslösen eines Alarmes und/oder automatische Abschaltung).

b) Änderung mindestens eines Prozessparameters, sodass die geeignete Kenngröße sinkt. Mögliche Änderungen sind dabei beispielsweise:

ba) Zufuhr von frischem Katalysator;

bb) Erhöhung der zurückgeführten Menge an gebrauchtem Katalysator;

bc) Graduelle Verringerung der eingespeisten Menge an frischem Nitroaromaten;

bd) Erhöhung des Drucks in mindestens einem der Reaktionsräume;

be) Erhöhung der Temperatur in mindestens einem der Reaktionsräume.

Es ist bevorzugt, die Menge an im Zulauf zum adiabat betriebenen Reaktionsraum gelöstem und dispergiertem Wasserstoff möglichst nicht zu stark absinken zu lassen. Unter dispergierten Wasserstoff wird dabei in Gasblasen mitgerissener Wasserstoff verstanden. Bevorzugt wird dabei in den Reaktionsraum soviel Wasserstoff nachgespeist, dass sich ein konstanter Druck einstellt, sowie ein kleiner Gasstrom aus der Gasphase des isothermen und des adiabaten Reaktionsraumes entnommen und ggf. auf seine Zusammensetzung hin analysiert wird (z. B. durch Gaschromatographie). Auf diese Weise wird umgesetzter Wasserstoff ergänzt und es werden sich ansammelnde inerte Gase oder gasförmige Reaktionsprodukte ausgeschleust.

Für das erfindungsgemäße Verfahren wesentlich ist nun, dass die Hydrierreaktion im adiabat betriebenen Reaktionsraum überhaupt vervollständigt werden kann. Das erfordert, dass die Menge an dispergiertem und gelöstem Wasserstoff im Zulauf zum adiabat betriebenen Reaktionsraum die für die Reaktion erforderliche Menge übersteigt. Dem Fachmann ist dabei bekannt, dass die Menge an gelöstem Wasserstoff von der Zusammensetzung des Reaktionsgemisches, dem Druck und der Temperatur im isotherm betriebenen Reaktionsraum sowie der Zusammensetzung der Gasphase in diesem Reaktionsraum abhängt. Für den Gehalt an dispergiertem und gelöstem Wasserstoff sind unter anderem die Begasung im Reaktionsraum sowie die Relativgeschwindigkeit von Flüssig- und Gasphase von Bedeutung. Die Zusammensetzung der Gasphase wird dabei durch die Partialdrücke der Komponenten in der flüssigen Phase und deren Wechselwirkung untereinander, der Bildung von gasförmigen Nebenprodukten wie z. B. Ammoniak sowie der Anwesenheit von Verunreinigungen im zugeführten Wasserstoff sowie deren Akkumulation im Reaktor bestimmt.

Weiterhin wesentlich ist, dass die flüssige Phase im adiabat betriebenen Reaktionsraum eine ausreichend große Verweilzeit aufweist, um eine Restumsetzung zu ermöglichen. Für das erfindungsgemäße Verfahren besitzt der adiabat betriebene Reaktionsraum eine Verweilzeit der Flüssigphase, die mindestens 10 % der Verweilzeit der Flüssigphase des isotherm betriebenen Reaktionsraums beträgt, dabei sind mindestens 20 % und höchstens 300 % bevorzugt.

Das erfindungsgemäße Verfahren ist besonders gut im Falle einer zu erwartenden graduellen und langsam stattfindenden Verringerung der Reaktionsgeschwindigkeit aus einem Zustand hoher Reaktionsgeschwindigkeit heraus geeignet, wie sie typischerweise, aber nicht ausschließlich, durch eine fortschreitende Katalysatordesaktivierung hervorgerufen wird. Der dem isotherm betriebenen Reaktionsraum nachgeschaltete adiabat betriebene Reaktionsraum ermöglicht aufgrund der höheren Temperaturen (keine Wärmeabfuhr!) höhere Umsätze, wodurch die Katalysatordesaktivierung mindestens zum Teil wieder ausgeglichen wird. Dazu müssen aber natürlich die sonstigen Vorraussetzung für einen weiteren Reaktionsfortschritt wie ausreichend Wasserstoff und Katalysator gegeben sein. Optional können daher Wasserstoff und Katalysator separat in den adiabat betriebenen Reaktionsraum zudosiert werden.

Die Aufarbeitung des vom Katalysator befreiten Produktgemisches umfasst zunächst die Abtrennung des bei der Hydrierung der Nitrogruppen gebildeten Reaktionswassers durch dem Fachmann bekannte destillative Auftrennung. Daran schließt sich die Abtrennung von Nebenprodukten (z. B. partiell oder vollständig kernhydrierte und/oder desaminierte und/oder demethylierte Nitroaromaten, Ammoniak (Desaminierungsprodukt), ggf. Reaktionsprodukte des Lösungsmittels (falls sich dieses nicht *vollständig* inert verhält), Azoxyverbindungen etc.) an. Die gewünschten aromatischen Amine werden von höhersiedenden Verunreinigungen (Rückstand) getrennt und erforderlichenfalls noch in ihre Isomere aufgetrennt. Diese Trennoperationen geschehen bevorzugt durch dem Fachmann bekannte Destillation.

Figur 1 zeigt eine Ausführungsform der Erfindung. Strom 1 bezeichnet das zu hydrierende Eduktgemisch (Nitroaromat, und ggf. inertes Lösungsmittel). Bevorzugt werden Nitroaromat und Katalysator getrennt zugegeben; der genaue Ort der Katalysatorzugabe (in Figur 1 nicht dargestellt) ist nicht erfindungswesentlich. Strom 2 bezeichnet Wasserstoff, A den Reaktor mit isotherm betriebenem Reaktionsraum, Strom 3 das in den Reaktor B mit adiabat betriebenem Reaktionsraum eintretende Reaktionsgemisch. Strom 4 bezeichnet das in die Filtration C (hier Querstromfilter) eintretende ausreagierte Gemisch, mit Strom 5 wird das eingedickte Gemisch aus Reaktionsprodukt und Katalysator in den Reaktor zurückgeführt, Strom 6 bezeichnet das Reaktionsprodukt, das der Aufarbeitung zugeführt wird und Strom 7 den der Ausschleusung von Inertgasen dienenden Gasstrom. Die Pumpe D dient dabei der Aufrechterhaltung des Kreislaufs. Die Überwachung der Vollständigkeit der Reaktion kann dabei beispielsweise durch Messung der Temperaturdifferenz $\Delta T_{adiabat}$ zwischen den Strömen 3 und 5, 3 und 4 oder 3 und 6 erfolgen.

Figur 2 stellt eine Ausführungsform dar, bei der die Funktionen des Reaktors B mit adiabat betriebenem Reaktionsraum und der Katalysatorabtrennung C in einem Apparat E zusammengefasst wird.

Figur 3 beschreibt eine Ausführungsform der Erfindung, bei der auf die Pumpe D verzichtet werden kann und die Rückführung des eingedickten Reaktionsgemischs aus dem Apparat E mit adiabat betriebenem Reaktionsraum mit Vorrichtung zur Katalysatorabtrennung (hier Schwerkraftabscheider) in den Reaktor A mit isotherm betriebenem Reaktionsraum mittels der Dichteunterschiede der flüssigen Phasen erfolgt.

Figur 4 beschreibt eine Ausführungsform, bei welcher der Reaktor A', der einen isotherm betriebenen Reaktionsraum A1 enthält, durch geeignete Strömungsführung zusätzlich noch einen adiabat betriebenen Reaktionsraum A2 enthält. Die Trennung der beiden Reaktionsräume kann dabei durch Einbauten (G), beispielsweise durch Siebböden, Leitbleche, Wärmeaustauscher oder Ähnliches erfolgen. Die Strömung im unteren Bereich des Reaktors ist turbulent und vergleichmäßigt sich nach oben hin zunehmend, insbesondere hinter G. Bei dieser Anordnung besteht der *insgesamt* adiabat betriebene Reaktionsraum aus zwei einzelnen adiabat betriebenen Reaktionsräumen, nämlich aus dem flüssigen Reaktorinhalt des Apparates E und einem Teil des Volumens des intensiv begasten Reaktors A', namentlich A2. Diese Anordnung hat den Vorteil eines besonders hohen Gasangebots im ersten adiabat betriebenen Reaktionsraum (A2) und ermöglicht somit die Umsetzung eines größeren Anteils an Nitroaromaten (z. B. Dinitrotoluol) und somit auch eine größere adiabate Temperaturdifferenz $\Delta T_{adiabat}$ zwischen E und A1, was die Messaufgabe weiter erleichtert. Die in A1 eingebrachten Gasblasen bewegen sich durch den adiabat betriebenen Reaktionsraum A2, sodass wesentlich mehr Nitro- und Nitrosogruppen hydiert werden können als in E allein. $\Delta T_{adiabat}$ kann bei dieser Anordnung bestimmt werden durch Messung der Temperatur in der Nähe des Rührers A1 und der Temperatur des aus Apparat E austretenden Produktstroms 5.

Figur 5 zeigt einen quantitativen Zusammenhang zwischen dem adiabaten Temperatursprung der Hydrierung von DNT und der Konzentration an Nitrotoluidin, die mindestens im Eintrittsstrom des adiabat betriebenen Reaktionsraums vorhanden ist (siehe Beispiel 2).

## Beispiele

### Beispiel 1 (erfindungsgemäß)

Einer Versuchsanordnung nach Figur 1 bestehend aus einem Reaktor A mit isotherm betriebenem Reaktionsraum mit 1 1 Reaktionsvolumen und innenliegendem Wärmeaustauscher, einem Reaktor B mit adiabat betriebenem Reaktionsraum mit einem Volumen von 1 1 sowie einer Vorrichtung zur Katalysatorabtrennung bestehend aus einer Umwälzpumpe (D) mit Fritte (C) wird Dinitrotoluol (Isomerengemisch aus 2,4-, 2,6-, 2,3-, 3,4-, 2,5-DNT) hydriert. Der Umlaufstrom 5 von 2,5 l/h fördert den in Apparat C vom Strom 6 abgetrennten Katalysator in den Reaktor A zurück. Der Wasserstoffstrom 2 wird so eingestellt, dass sich in beiden Reaktionsräumen jeweils ein absoluter Druck von 25 bar ergibt. Ca. 4 Vol.-% des zugeführten Wasserstoffs werden zwecks Ausschleusung von Verunreinigungen und gasförmigen Nebenprodukten über Strom 7 abgeführt. Das ausreagierte Gemisch Strom 6 wird gaschromatographisch analysiert. Durch den Wärmeaustauscher wird Kühlwasser geführt, dessen Menge so eingestellt wird, dass sich eine Temperatur im Reaktionsraum von A von 120 °C einstellt. Es werden insgesamt 40 g Raney-Ni in Wasser suspendiert als

Hydrierkatalysator zugegeben. Die Temperaturen in Strom 4 und im Reaktionsraum in der Nähe des Rührers werden gemessen, ihre Differenz errechnet und aufgezeichnet. Die Reaktion wird in Betrieb genommen, indem ein Strom von 400 g/h Dinitrotoluol (1) zugeführt wird. Es wird eine Temperaturdifferenz $\Delta T_{adiabat}$ = T(4) - T(A, Rührer) = -1 K gemessen (d. h. in B findet keine Reaktion statt), während in Strom 6 keine Nitroaromaten mittels Gaschromatographie ermittelt werden können. Die Reaktion wird so lange unter ansonsten konstanten Bedingungen durchgeführt, bis die Temperaturdifferenz $\Delta T_{adiabat}$ auf +8 K angestiegen ist (d. h. in A wird das DNT nicht mehr vollständig umgesetzt). Dies ist nach 360 Std. erreicht. Dann wird die Reaktion abgebrochen und ein Gehalt von 3,8 Massen % Nitrotoluidine im Strom 6 gefunden.

**Beispiel 2 (erfindungsgemäß)**

Anhand der Reaktionswärme für die Hydrierreaktion und Wärmekapazität des bei der Reaktion aus Beispiel 1 gebildeten Stoffgemisches wird der in Figur 5 gezeigte Zusammenhang zwischen $\Delta T_{adiabat}$ = T(4) - T(A, Rührer) und der im Strom 3 zum adiabat betriebenen Reaktionsraum gefundenen Konzentration am Zwischenprodukt Nitrotoluidin ($c_{NT}$; angegeben in Massen-% bezogen auf die Gesamtmasse des Stromes 3) bestimmt. Dabei stellt $c_{NT}$ die *Mindest*konzentration an Nitrotoluidin unter der Annahme perfekter Wärmeisolation dar. Der in Figur 5 dargestellte Zusammenhang beruht ferner auf der Annahme, dass im adiabat betriebenen Reaktionsraum stets ausreichend Wasserstoff für die vollständige Abreaktion von Nitrogruppen vorhanden ist. Zur Erstellung von Figur 5 (oder vergleichbaren Diagrammen) erforderliche Stoffdaten können beispielsweise der dem Fachmann geläufigen Datenbank DIPPR entnommen werden. Bei $\Delta T_{adiabat} \geq$ +3 K wird frischer Katalysator zugegeben, um sicherzustellen, dass der adiabat betriebene Reaktionsraum nicht überlastet wird.

**Beispiel 3 (erfindungsgemäß)**

In der Anordnung aus Beispiel 1 werden 15 g Katalysator, in Wasser suspendiert, eingewogen und die Reaktion wird begonnen. Jedes Mal, wenn $\Delta T_{adiabat}$ einen Wert von 1 K überschreitet, werden 3 g Katalysator hinzugefügt. Das aus dem adiabat betriebenen Reaktionsraum austretende Reaktionsprodukt (Strom 4) wird regelmäßig gaschromatographisch untersucht; DNT, Nitrotoluidine und Nitrosoverbindungen werden nicht gefunden.

**Patentansprüche**

1. Verfahren zur Herstellung von aromatischen Aminen der Formel

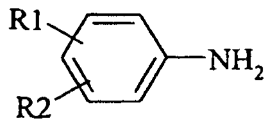

(I)

in der R1 und R2 unabhängig voneinander Wasserstoff, Methyl oder Ethyl bedeuten, wobei R1 zusätzlich $NH_2$ bedeuten kann, durch Hydrierung in Gegenwart eines Katalysators von Nitroaromaten der Formel

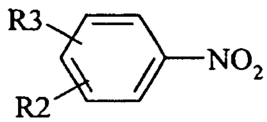

(II)

in der R2 und R3 unabhängig voneinander Wasserstoff, Methyl oder Ethyl bedeuten, wobei R3 zusätzlich $NO_2$ bedeuten kann, in der Flüssigphase in mindestens zwei hintereinandergeschalteten, in einem Reaktor oder in mehreren Reaktoren angeordneten Reaktionsräumen, wobei
mindestens ein Reaktionsraum isotherm und mindestens der diesem nachgeschaltete Reaktionsraum adiabat betrieben wird.

2. Verfahren nach Anspruch 1, wobei der Reaktionsverlauf der Hydrierung durch Kontrolle einer für den in mindestens einem der adiabat betriebenen Reaktionsräume durchgeführten Teil der Hydrierung charakteristischen Kenngröße

überwacht wird und bei Erreichen oder Überschreiten eines kritischen Wertes dieser Kenngröße mindestens ein Prozessparameter so geändert wird, dass diese Kenngröße wieder unter den kritischen Wert sinkt, wobei die geeignete Kenngröße ausgewählt ist aus der Gruppe bestehend aus

der Temperaturdifferenz $\Delta T_{adiabat} = T_{adiabat} - T_{isotherm}$ zwischen mindestens einem der adiabat betriebenen Reaktionsräume und dem diesen jeweils vorangehenden isotherm betriebenen Reaktionsraum, wobei $T_{adiabat}$ im Produktaustritt aus dem jeweiligen adiabat betriebenen Reaktionsraum oder im jeweiligen adiabat betriebenen Reaktionsraum und $T_{isotherm}$ im jeweils vorangehenden isotherm betriebenen Reaktionsraum oder im Eintritt in den jeweiligen adiabat betriebenen Reaktionsraum gemessen werden, und wobei der kritische Wert der Kenngröße $\Delta T_{adiabat}$ ($\Delta T_{adiabat,\ krit.}$) zwischen -10 K und +8 K liegt;

und

der Konzentration an Nitro- und Nitrosogruppen enthaltenden Verbindungen, wobei der kritische Wert für die Kenngröße Konzentration an Nitro- und Nitrosogruppen enthaltenden Verbindungen bis kleiner als 5,0 Massen-%, bezogen auf die Gesamtmasse des Produktaustrittstroms des adiabat betriebenen Reaktionsraumes, beträgt.

**3.** Verfahren nach Anspruch 2, wobei die Konzentration an Nitro- und Nitrosogruppen enthaltenden Verbindungen durch chemische oder spektroskopische Analyse ermittelt wird.

**4.** Verfahren nach einem der Ansprüche 2 bis 3, wobei die Änderung des mindestens einen Prozessparameters ausgewählt ist aus den Möglichkeiten:

a) Zufuhr von frischem Katalysator;
b) Erhöhung der zurückgeführten Menge an gebrauchtem Katalysator;
c) Graduelle Verringerung der eingespeisten Menge an frischem Nitroaromaten;
d) Erhöhung des Drucks in mindestens einem der Reaktionsräume;
e) Erhöhung der Temperatur in mindestens einem der Reaktionsräume.

**5.** Verfahren nach einem der Ansprüche 1 bis 4, wobei in allen Reaktionsräumen jeweils Temperaturen von 50 °C bis 250 °C und absolute Drücke von 6 bar bis 101 bar eingehalten werden.

**6.** Verfahren nach Anspruch 5, wobei das Verhältnis aus dem Druck in einem beliebigen adiabat betriebenen Reaktionsraum zum Druck im diesen jeweils vorangehenden isotherm betriebenen Reaktionsraum größer als 0,5 ist.

**7.** Verfahren nach einem der Ansprüche 1 bis 6, wobei Dinitrotoluol (in Formel II: R2 = Methyl und R3 = $NO_2$) zu Toluyendiamin (in Formel I: R1 = $NH_2$ und R2 = Methyl) hydriert wird.

**8.** Verfahren nach einem der Ansprüche 1 bis 7, wobei zwischen 2 und 10 Reaktionsräume hintereinandergeschaltet werden.

**9.** Verfahren nach einem der Ansprüche 1 bis 8, wobei der in Anströmrichtung des Nitroaromaten erste Reaktionsraum isotherm und der letzte adiabat betrieben werden.

## Claims

**1.** Process for the preparation of aromatic amines of formula

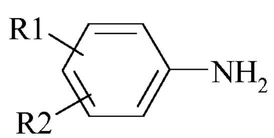

(I)

in which R1 and R2 independently of one another denote hydrogen, methyl or ethyl, wherein R1 can additionally denote $NH_2$, by hydrogenation, in the presence of a catalyst, of nitroaromatic compounds of formula

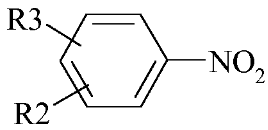

(II)

in which R2 and R3 independently of one another denote hydrogen, methyl or ethyl, wherein R3 can additionally denote $NO_2$, in the liquid phase in at least two series-connected reaction spaces arranged in one reactor or in a plurality of reactors, wherein
at least one reaction space is operated isothermally and at least the reaction space connected downstream thereof is operated adiabatically.

2. Process according to claim 1, wherein the progress of the hydrogenation reaction is monitored by monitoring a parameter characteristic of the part of the hydrogenation that is carried out in at least one of the adiabatically operated reaction spaces and, when a critical value of that parameter is reached or exceeded, at least one process parameter is so changed that the parameter falls below the critical value again, the suitable parameter being selected from the group consisting of the temperature difference $\Delta T_{adiabatic} = T_{adiabatic} - T_{isothermal}$ between at least one of the adiabatically operated reaction spaces and the isothermally operated reaction space preceding it, $T_{adiabatic}$ being measured in the product outlet from the adiabatically operated reaction space or in the adiabatically operated reaction space and $T_{isothermal}$ being measured in the preceding isothermally operated reaction space or in the inlet into the adiabatically operated reaction space, and the critical value of the parameter $\Delta T_{adiabatic}$ ($\Delta T_{adiabatic, crit.}$) being from -10 K to +8 K;
and the concentration of compounds containing nitro and nitroso groups, the critical value for the parameter concentration of compounds containing nitro and nitroso groups being up to less than 5.0% by mass, based on the total mass of the product outlet stream of the adiabatically operated reaction space.

3. Process according to claim 2, wherein the concentration of compounds containing nitro and nitroso groups is determined by chemical or spectroscopic analysis.

4. Process according to any one of claims 2 to 3, wherein the change of the at least one process parameter is selected from the possibilities:

a) supply of fresh catalyst;
b) increase in the amount of used catalyst supplied;
c) gradual reduction in the amount of fresh nitroaromatic compound fed in;
d) increase in the pressure in at least one of the reaction spaces;
e) increase in the temperature in at least one of the reaction spaces.

5. Process according to any one of claims 1 to 4, wherein temperatures of from 50°C to 250°C and absolute pressures of from 6 bar to 101 bar are maintained in all the reaction spaces.

6. Process according to claim 5, wherein the ratio of the pressure in an arbitrary adiabatically operated reaction space to the pressure in the isothermally operated reaction space preceding it is greater than 0.5.

7. Process according to any one of claims 1 to 6, wherein dinitrotoluene (in formula II: R2 = methyl and R3 = $NO_2$) is hydrogenated to toluylenediamine (in formula I: R1 = $NH_2$ and R2 = methyl).

8. Process according to any one of claims 1 to 7, wherein from 2 to 10 reaction spaces are connected in series.

9. Process according to any one of claims 1 to 8, wherein the first reaction space in the direction of flow of the nitroaromatic compound is operated isothermally and the last is operated adiabatically.

**Revendications**

1. Procédé pour la préparation d'amines aromatiques de formule

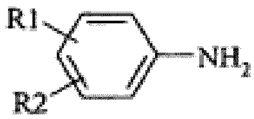

(I)

dans laquelle R1 et R2 représentent indépendamment l'un de l'autre un atome d'hydrogène, le groupe méthyle ou éthyle, R1 pouvant en outre représenter $NH_2$, par hydrogénation en présence d'un catalyseur de composés nitro-aromatiques de formule

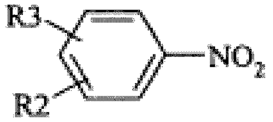

(II)

dans laquelle R2 et R3 représentent indépendamment l'un de l'autre un atome d'hydrogène, le groupe méthyle ou éthyle, R3 pouvant en outre représenter $NO_2$, dans la phase liquide, dans au moins deux espaces réactionnels raccordés en série, disposés dans un réacteur ou dans plusieurs réacteurs,
au moins un espace réactionnel étant utilisé en mode isothermique et au moins l'espace réactionnel raccordé en aval à celui-ci étant utilisé en mode adiabatique.

2. Procédé selon la revendication 1, dans lequel on surveille le déroulement de la réaction de l'hydrogénation par contrôle d'une grandeur caractéristique de la partie de l'hydrogénation qui est effectuée dans au moins un des espaces réactionnels utilisés en mode adiabatique et lorsqu'une valeur critique de cette grandeur caractéristique est atteinte ou excédée on modifie au moins un paramètre de processus de manière que cette grandeur caractéristique s'abaisse de nouveau au-dessous de la valeur critique, la grandeur caractéristique appropriée étant choisie dans le groupe constitué par la différence de température $\Delta T_{adiabat.} = T_{adiabat.} - T_{isotherm.}$ entre au moins un des espaces réactionnels utilisés en mode adiabatique et l'espace réactionnel utilisé en mode isothermique, le précédant chaque fois, $T_{adiabat}$. étant mesurée à la sortie du produit de l'espace réactionnel respectif utilisé en mode adiabatique ou dans l'espace réactionnel respectif utilisé en mode adiabatique et $T_{isotherm.}$ étant mesurée dans l'espace réactionnel respectif précédant utilisé en mode isothermique ou à l'entrée dans l'espace réactionnel respectif utilisé en mode adiabatique, et la valeur critique de la grandeur caractéristique $\Delta T_{adiabat.}$ ($\Delta T_{adiabat.\ crit.}$) étant comprise entre -10 K et +8 K ;
et
la concentration de composés contenant des groupes nitro et nitroso, la valeur critique de la grandeur caractéristique concentration de composés contenant des groupes nitro et nitroso valant jusqu'à moins de 5,0 % en masse, par rapport à la masse totale du courant de sortie du produit de l'espace réactionnel utilisé en mode adiabatique.

3. Procédé selon la revendication 2, dans lequel la concentration de composés contenant des groupes nitro et nitroso est déterminée par analyse chimique ou spectroscopique.

4. Procédé selon l'une quelconque des revendications 2 et 3, dans lequel la modification dudit au moins un paramètre de processus est choisie parmi les possibilités:

   a) Introduction de catalyseur neuf;
   b) Augmentation de la quantité recyclée de catalyseur utilisé ;
   c) Diminution graduelle de la quantité introduite de composé nitro-aromatique neuf ;
   d) Élévation de la pression dans au moins un des espaces réactionnels.
   e) Élévation de la température dans au moins un des espaces réactionnels.

5. Procédé selon l'une quelconque des revendications 1 à 4, dans lequel on maintient dans tous les espaces réactionnels respectivement des températures de 50 °C à 250 °C et des pressions absolues de 6 bars à 101 bars.

6. Procédé selon la revendication 5, dans lequel le rapport de la pression dans un quelconque espace réactionnel utilisé en mode adiabatique à la pression dans l'espace réactionnel utilisé en mode isothermique le précédant chaque fois est supérieur à 0,5.

7. Procédé selon l'une quelconque des revendications 1 à 6, dans lequel on convertit par hydrogénation du dinitrotoluène (dans la formule II: R2 = méthyle et R3 = $NO_2$) en toluylènediamine (dans la formule I : R1 = $NH_2$ et R2 = méthyle).

8. Procédé selon l'une quelconque des revendications 1 à 7, dans lequel entre 2 et 10 espaces réactionnels sont raccordés en série.

9. Procédé selon l'une quelconque des revendications 1 à 8, dans lequel, dans le sens de l'écoulement du composé nitro-aromatique, le premier espace réactionnel est utilisé en mode isothermique et le dernier est utilisé en mode adiabatique.

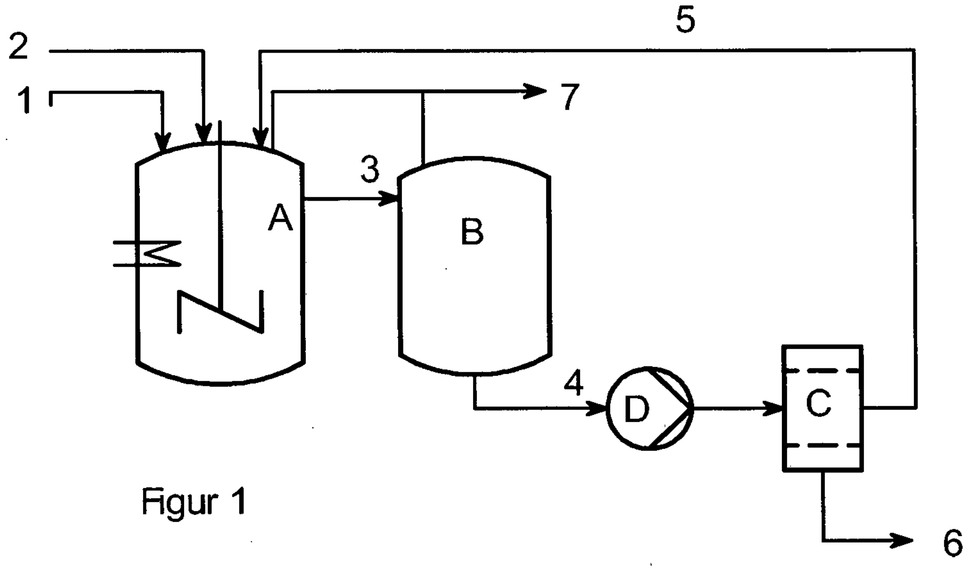


Figur 1

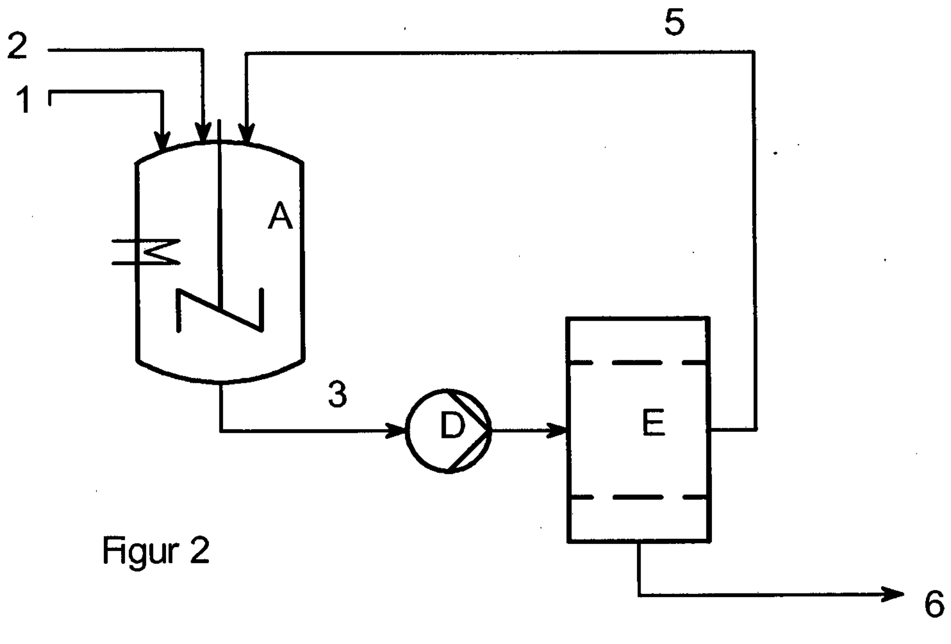


Figur 2

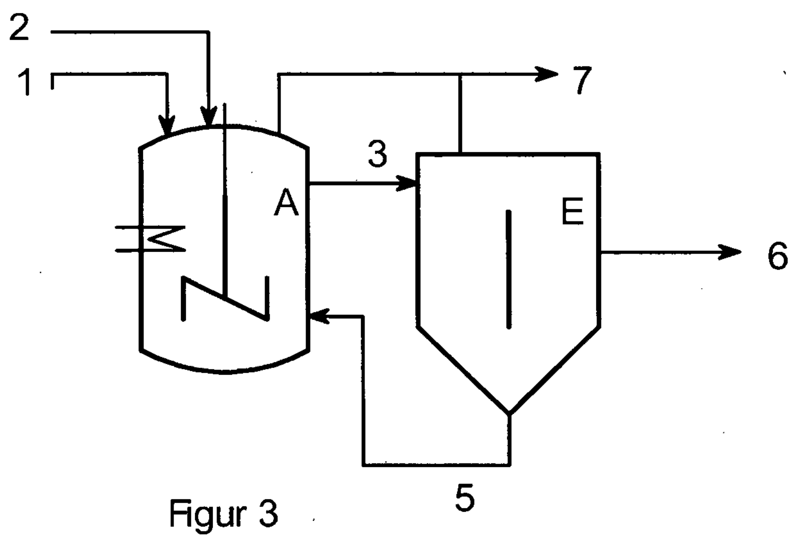


Figur 3

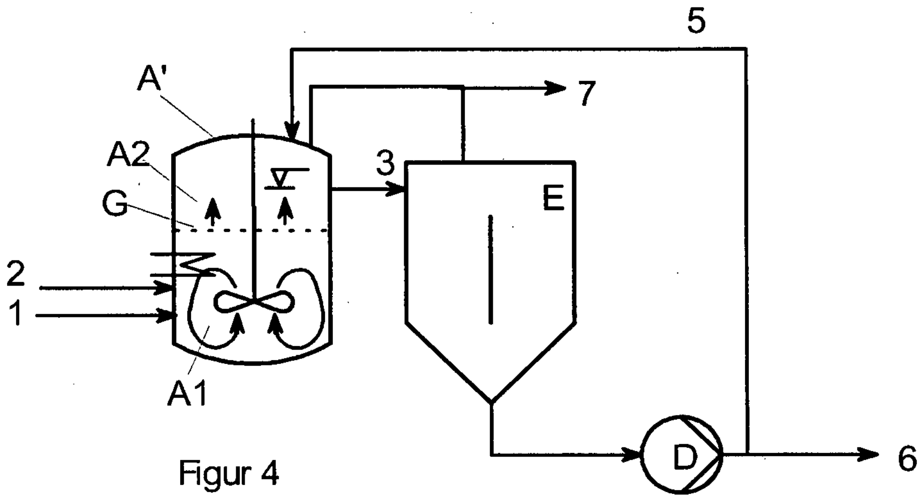


Figur 4

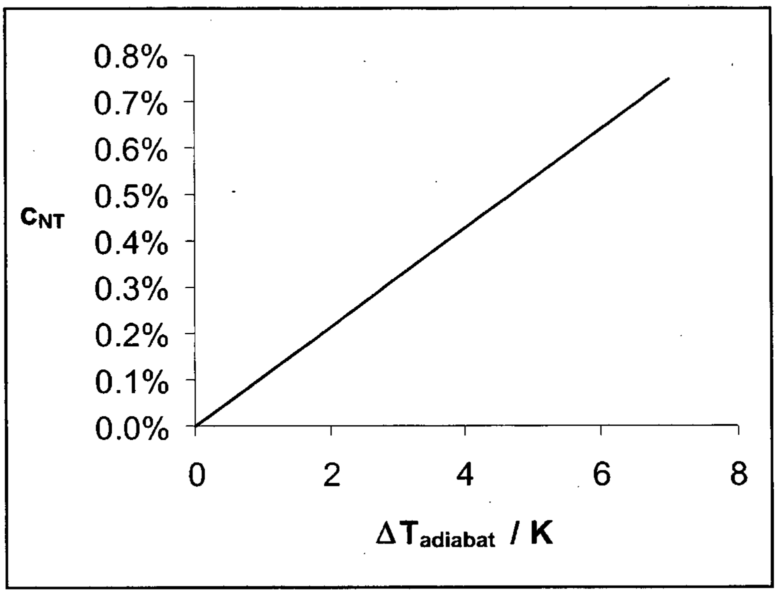


Figur 5

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0223035 A1
- WO 9611052 A1
- DE OS2044657 A
- US 3761521 A
- DE 19844901 C1
- EP 0634391 A1
- WO 0035852 A1
- WO 0035852 A
- DE 10206214 A1
- WO 03066571 A1
- DE 102005008613 A1
- WO 02062729 A2
- EP 1935871 A
- EP 1524259 A1
- GB 1452466 A